(19) Europäisches Patentamt
European Patent Office
Office européen des brevets

(11) Numéro de publication: **0 172 059 B1**

# (12) FASCICULE DE BREVET EUROPEEN

(45) Date de publication du fascicule du brevet: **20.04.88**

(51) Int. Cl.⁴: **C 07 F 9/65**, A 61 K 31/675

(21) Numéro de dépôt: **85401359.6**

(22) Date de dépôt: **04.07.85**

(54) **Nouveaux dérivés de l'oxaazaphosphorine, leur procédé de préparation et les compositions pharmaceutiques les renfermant.**

(30) Priorité: **06.07.84 FR 8410731**

(43) Date de publication de la demande: **19.02.86 Bulletin 86/8**

(45) Mention de la délivrance du brevet: **20.04.88 Bulletin 88/16**

(84) Etats contractants désignés: **AT BE CH DE FR GB IT LI LU NL SE**

(56) Documents cités:
**FR - A - 2 536 075**
**PATENTS ABSTRACTS OF JAPAN, vol. 8, no. 118 (C-226) [1555], 31 mai 1984**

(73) Titulaire: **ADIR, 22, rue Garnier, F-92200 Neuilly-sur-Seine (FR)**

(72) Inventeur: **Lavielle, Gilbert, 1 avenue Lilly, F-78170 La Celle St Cloud (FR)**
Inventeur: **Cudennec, Claude, 23 bis, av. de Circourt, F-78170 La Celle St Cloud (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 172 059 B1

ACTORUM AG

**Description**

La présente invention concerne de nouveaux dérivés de l'oxaazaphosphorine, leur procédé de préparation et les compositions pharmaceutiques les contenant.

Des oxaazaphosphorines sont connues dans la littérature et utilisées en thérapeutique, principalement en chimiothérapie anti-cancéreuse et l'une des drogues anti-tumolares les plus largement utilisées, le cyclophosphamide (Angew. Chem. 70, 539, 1958), est une moutarde à l'azote $[-N(CH_2-CH_2-Cl)_2]$ directement greffée sur l'atome de phosphore d'un hétérocycle oxaazaphosphorine. Cet hétérocycle joue un rôle non seulement comme vecteur de l'agent alkylant, mais aussi un rôle biologique important et complexe dans l'activation de la molécule.

Les demandes de brevet japonais 141 308 (13.08.1982) et 141 309 (13.08.1982) décrivent des oxo-2 (N-nitroso halo-2 éthylamino)-2 oxaazaphosphorines dans lesquelles les propriétés de l'hétérocycle sont utilisées comme vecteur d'un autre reste alkylant, fixé lui-aussi sur l'atome de phosphore.

D'autres agents alkylants, les nitrosourées sont également utilisées en thérapeutique anti-cancéreuse, mais on connaît la décomposition spontanée en solution des nitrosourées monosubstituées sur l'atome d'azote, après leur activation par arrachement du proton qu'elles portent sur l'atome d'azote. Par contre, les nitrosourées disubstituées sur l'atome d'azote sont stables en solution mais en général inactives (J. Med. Chem., 27, 97, 1984).

L'objet de la présente invention est de rechercher des nitrosourées stables en solution, mais également actives in vivo et in vitro.

Le but est atteint lorsque l'on prépare les dérivés de l'oxo-2 tétrahydro-2H oxaazaphosphorine-1,3,2 répondant à la formule générale I:

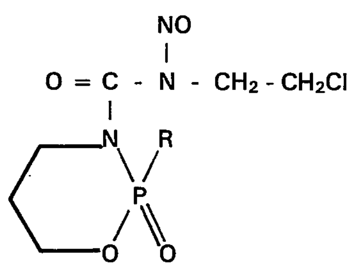

I

dans laquelle R représente:

- un groupe alkyle en chaîne droite ou ramifiée comportant de 1 à 6 atomes de carbone,
- un groupe cycloalkyle comportant de 3 à 7 atomes de carbone,
- un groupe phényle pouvant être substitué par un radical alkyle ou alcoxy comportant de 1 à 4 atomes de carbone ou par un atome d'halogène,
- un groupe phénylalkyle comportant de 7 à 10 atomes de carbone dans lequel le noyau phényle peut-être éventuellement substitué par un atome d'halogène ou un radical alkyle ou alcoxy de 1 à 4 atomes de carbone,
- un groupe alcoxy de 1 à 4 atomes de carbone en chaîne droite ou ramifiée,
- un groupe phénoxy ou thiophénoxy,
- un groupe mono- ou di-alkylamino comportant de 1 à 8 atomes de carbone pouvant être éventuellement substitué par un ou deux atomes d'halogène, ou
- un groupe pyrrolidinyle-1, morpholinyle-4, pipéridinyle-1 ou un groupe pipérazinyle-1 éventuellement substitué en position 4 par un reste alkyle de 1 à 4 atomes de carbone en chaîne droite ou ramifiée, par un groupe cycloalkyle de 5 à 7 atomes de carbone, ou par un groupe phényle ou phénylalkyle de 7 à 10 atomes de carbone dans lesquels le noyau aromatique peut être substitué par un atome d'halogène ou un radcial alkyle ou alcoxy comportant de 1 à 4 atomes de carbone en chaîne droite ou ramifiée,

ainsi que, lorsque R représente un groupe diaminé, leurs sels d'addition à des acides pharmaceutiquement acceptables (chlorhydrates, sulfates, méthanesulfonates, oxalates, fumarates, maléates...).

Les composés actuellement préférés sont les dérivés de formule générale I dans laquelle R représente un groupe aminé et en particulier un radical pyrrolidinyle, pipéridinyle, morpholinyle ou pipérazinyle éventuellement substitué.

La présente invention a également pour objet la préparation des composés de formule I, caractérisé en ce que l'on condense un dichlorure d'acide phosphonique ou phosphorique de formule II

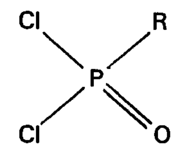

II

dans laquelle R a la même signification que dans la formule I,

avec l'amino-3 propanol-1 dans un solvant inerte et en présence d'un accepteur d'acide à une température comprise entre −30 et 0 °C, en un composé de formule III:

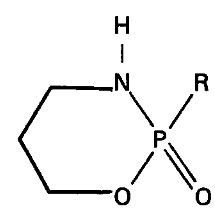

III

dans laquelle R a la même signification que dans la formule I,

que l'on condense avec un excès de β-chloroéthyl isocyanate de formule IV:

$$Cl-CH_2-CH_2-N=C=O \quad IV$$

soit directement lorsque R représente un groupe aminé, soit, quant R est un groupe non-aminé, après activation préalable de l'atome d'azote du cycle oxaazaphosphorine-1,3,2 par le butyllithium dans un solvant basique comme le tétrahydrofuranne à une température comprise entre −70 et −40 °C, en un composé de formule V:

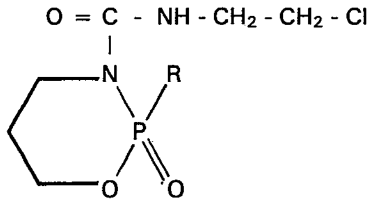

V

dans laquelle R a la même signification que dans la formule I,

qui est ensuite nitrosé en milieu acide ou basique par action d'agents nitrosants comme le chlorure de nitrosyle, le nitrile de sodium, le tétroxyde d'azote, etc. en un composé de formule I,

qui peut-être ensuite transformé, lorsque R est un groupe diaminé, en son sel d'addition à un acide pharmaceutiquement acceptable.

Pour réaliser la nitrosation, il est avantageux d'activer préalablement les composés de formule V en les traitant par exemple par un équivalent de butyllithium dans le tétrahydrofuranne à une température de −80°C.

Les matières premières de formule II ont été préparées suivant des méthodes décrites dans la littérature, et en particulier dans:

Methoden der Organischen Chemie.
Houben Weyl - Vierte Auflage Phosphor Verbindungen I et II
Georg Thieme Verlag 1963.

Les exemples suivants illustrent l'invention et ne la limitent en aucune façon. Les points de fusion ont été déterminés sur une plaque chauffante selon Kofler. Les spectres RMN ont été obtenus en utilisant le tétraméthylsilane comme référence interne et le chloroforme deutérié comme solvant. Les caractéristiques physico-chimiques et spectrales des composés mentionnés à titre d'exemples sont rassemblés dans le tableau I.

*Exemple 1*

[N (chloro-2 éthyl) N-nitrosamido] -3 isopropyl-2 oxo-2 tétrahydro-2H oxaazaphosphorine-1,3,2

*Stade A:* Isopropyl-2 oxo-2 tétrahydro-2H oxaazaphosphorine-1,3,2.

A une solution de 0,1 mole d'amino-3 propanol-1 et de 0,2 mole de triéthylamine dans 200 $cm^3$ de dichlorométhane refroidi à −20°C, on ajoute sous agitation 0,1 mole de dichlorure de l'acide isopropyl phosphonique.

On agite le mélange réactionnel pendant 1 heure à température ambiante, filtre le précipité, et lave les eaux-mères par deux fois 50 $cm^3$ d'eau saturée de chlorure de sodium.

La phase organique est prélévée, séchée et évaporée, et l'on obtient 14,5 g d'isopropyl-2 oxo-2 tétrahydro-2H oxaazaphosphorine-1,3,2 qui est utilisée brute au stade suivant.

*Stade B:* [N-(chloro-2 éthyl) amido] -3 isopropyl-2 oxo-2 tétrahydro-2H oxaazaphosphorine-1,3,2.

Dans un récipient contenant 150 $cm^3$ de tétrahydrofuranne, on introduit les 14,5 grammes d'isopropyl-2 oxo-2 tétrahydro-2H oxaazaphosphorine--1,3,2 obtenus au stade précédent, ajoute au mélange refroidi à −80°C un équivalent de butyllithium en solution dans l'hexane et maintient la solution à cette température pendant deux heures. On ajoute en une seule fois deux équivalents de β-chloroéthyl isocyanate en solution dans 20 $cm^3$ de tétrahydrofuranne. On maintient la solution pendant quinze minutes à une température de −50°C, l'hydrolyse et l'extrait par 3 fois 100 $cm^3$ d'éther éthylique. Les solutions éthérées sont réunies, séchées et évaporées. Le résidu huileux obtenu est repris par un peu d'isopropanol et le précipité qui se forme, séparé par filtration. Les eaux-mères sont évaporées sous pression réduite, et les 14,5 grammes d'huile obtenus sont chromatographiés sur une colonne de 300 g de silice (solvant dichlorométhane-méthanol; 98-2).

On obtient 10 g de [N-(chloro-2 éthyl) amido] -3 isopropyl-2 oxo-2 tétrahydro-2H oxaazaphosphorine-1,3,2 (Rendement 42%).

Analyse centésimale:

| | | | | |
|---|---|---|---|---|
| Calculé: | C 40,23 | H 6,75 | N 10,43 | Cl 13,20 |
| Trouvé: | C 39,77 | H 6,76 | N 10,04 | Cl 13,26 |

pour $C_9H_{18}ClN_2O_3P$ = 268,5

Principales caractéristiques spectrales

– infra-rouge: ν NH: 3500 et 3300 $cm^{-1}$
ν C=O: 1680 et 1540 $cm^{-1}$

– résonance magnétique nucléaire:

1,1 ppm; 3H; d de d; JPH = $2H_z$; JHH = 6,3 Hz
1,4 ppm; 3H; d; JHH = 6,3 Hz
1,7 à 2,7 ppm; 3H,m
2,8 à 3,5 ppm; 1H,m
3,6 ppm; 4H; $A_2B_2$
4 à 4,8 ppm; 2H; m
8,1 ppm; 1H échangeable; m.

*Stade C:* [N-(chloro-2 éthyl) N-nitrosoamido] -3 isopropyl-2 oxo-2 tétrahydro-2H oxaazaphosphorine-1,3,2.

Dans un récipient contenant 50 $cm^3$ de tétrahydrofuranne, on introduit 5,2 grammes de [N-(chloro-2 éthyl) amido] -3 isopropyl-2 oxo-2 tétrahydro--2H oxaazaphosphorine-1,3,2 obtenus au stade précédent. On refroidit le mélange à −80°C puis ajoute goutte à goutte un équivalent de butyllithium en solution dans l'hexane et on maintient la solution à −80°C en agitant. On introduit alors rapidement un équivalent de chlorure de nytrosyle. La solution verte obtenue se décolore rapidement. A complète décoloration, on l'hydrolyse à froid et on l'extrait par 3 fois 50 $cm^3$ d'éther éthylique. On réunit les fractions éthérées, les sèche puis les évapore. L'huile obtenue (5,4 g) est rapidement chromatographiée sur colonne de silice (solvant dichlorométhane-acétone; 90-10) et le produit obtenu est cristallisé dans un peu d'éther de pétrole. On obtient 4,7 grammes de [N-(chloro-2 éthyl) N-nitrosoamido] -3 isopropyl-2 oxo-2 tétrahydro-2H oxaazaphosphorine-1,3,2 (Rendement 81%).

*Exemple 2*

[N-(chloro-2 éthyl) N-nitroso amido] -3 phényl-2 oxo-2 tétrahydro-2H oxaazaphosphorine-1,3,2

*Stade A:* Phényl-2 oxo-2 tétrahydro-2H oxaazaphosphorine-1,3,2.

En procédant selon le mode opératoire décrit dans l'exemple 1, stade A à partir de 0,41 mole de dichlorure de l'acide phénylphosphonique et d'un mélange de 0,41 mole d'amino-3 propanol-1 et de 0,82 mole de triéthylamine dans 800 $cm^3$ de dichlorométhane, on obtient 38 grammes de produit que l'on purifie par chromatographie sur colonne de silice (solvant = dichlorométhane-méthanol 98-2).

On obtient 30 grammes de phényl-2 oxo-2 tétrahydro-2H oxaazaphosphorine-1,3,2.

*Stade B:* [N-(chloro-2 éthyl) amido] -3 phényl-2 oxo-2 tétrahydro-2H oxaazaphosphorine-1,3,2.

On refroidit à −80°C une solution de 25,6 grammes (0,13 mole) de phényl-2 oxo-2 tétrahydro-2H oxaazaphosphorine-1,3,2 dans 300 $cm^3$ de tétrahydrofuranne et y introduit goutte à goutte un équivalent de butyllithium en solution dans l'hexane, agite pendant 30 minutes à −65°C, refroidit à nouveau à −80°C puis ajoute en une seule fois deux équivalents de β-chloroéthyl isocyanate en solution dans 25 $cm^3$ de tétrahydrofuranne. On maintient le mélange réactionnel pendant une demi-heure à −80°C, l'hydrolyse puis l'extrait par 3 fois 150 $cm^3$ d'éther éthylique. Les extraits éthérés sont réunis, séchés, évaporés, et le résidu huileux obtenu repris par un peu d'isopropanol. On essore le précipité et évapore les eaux-mères. On recueille 32 grammes de produit dont on purifie 12 grammes par chromatographie sur colonne de silice (solvant: cyclohexane-acétone; 70-37) pour obtenir 6,6 grammes de [N-(chloro-2 éthyl) amido] -3 phényl-2 oxo-2 tétrahydro-2H oxaazaphosphorine-1,3,2 purs.

*Stade C:* [N-(chloro-2 éthyl) N-nitrosoamido] -3 phényl-2 oxo-2 tétrahydro-2H oxaazaphosphorine-1,3,2.

Dans 75 $cm^3$ de tétrahydrofuranne, on introduit les 6,6 grammes de [N-(chloro-2 éthyl) amido] -3 phényl-2 oxo-2 tétrahydro-2H oxaazaphosphorine-1,3,2 obtenus au stade précédent, refroidit à −80°C et ajoute goutte à goutte un équivalent de butyllithium en solution dans l'hexane. On maintient le mélange à −80°C sous agitation pendant une heure, puis introduit rapidement un équivalent de chlorure de nitrosyle et ramène la température à −65°C en poursuivant l'agitation. On hydrolyse le mélange réactionnel, l'extrait par 3 fois 100 $cm^3$ d'éther éthylique. Les extraits éthérés réunis, séchés et concentrés abandonnent 7,9 grammes d'une huile jaune qui sont chromatographiés rapidement suivant la technique décrite par W.C. STILL (J. Org. Chem. 1978, 43, 2923) (solvant: éther-hexane-méthanol; 70-25-5). On recueille 4,5 grammes de [N-(chloro-2 éthyl) N-nitrosoamido] -3 phényl-2 oxo-2 tétrahydro-2H oxaazaphosphorine-1,3,2; qui sont cristallisés dans un peu d'éther diisopropylique (Rendement 62%).

*Exemple 3*

[N-(chloro-2 éthyl) N-nitrosoamido] -3 méthoxy-2 oxo-2 tétrahydro-2H oxaazaphosphorine-1,3,2

*Stade A:* Méthoxy-2 oxo-2 tétrahydro-2H oxaazaphosphorine-1,3,2.

En remplaçant dans l'exemple 1, stade A, le dichlorure de l'acide isopropyl phosphonique par le méthyl dichlorophosphate, on obtient la méthoxy-2 oxo-2 tétrahydro-2H oxaazaphosphorine-1,3,2 (Rendement 50%).

Eb (0,4 mm/Hg) = 140°C.

*Stade B:* [N-(chloro-2 éthyl) amido] -3 méthoxy-2 tétrahydro-2H oxaazaphosphorine-1,3,2.

En remplaçant dans l'exemple 2, stade B, la phényl-2 oxo-2 tétrahydro-2H oxaazaphosphorine-1,3,2 par la méthoxy-2 oxo-2 tétrahydro-2H oxaazaphosphorine-1,3,2, on obtient de la même façon la [N-(chloro-2 éthyl) amido] -3 méthoxy-2 oxo-2 tétrahydro-2H oxaazaphosphorine-1,3,2 (Rendement 58%) F° = 50°C.

Analyse centésimale:

| | | | | |
|---|---|---|---|---|
| Calculé: | C 32,75 | H 5,50 | N 10,91 | Cl 13,81 |
| Trouvé: | C 32,88 | H 5,41 | N 10,96 | Cl 13,65 |

pour $C_7H_{14}ClN_2O_4P$ = 256,632

Caractéristiques spectrales en:

- infra-rouge: ν NH: 3340 $cm^{-1}$
  ν C=O: 1680 et 1535 $cm^{-1}$
- RMN: 1,5 à 2,5 ppm; 2H; m
  2,5 à 3,5 ppm; 1H; m
  3,6 ppm; 4H; $A_2B_2$
  3,8 ppm; 3H; d; JPH = 12$H_z$
  4,5 ppm; 3H; m
  8 ppm; 1H; m; échangeable.

*Stade C:* [N-(chloro-2 éthyl) N-nitrosoamido] -3 méthoxy-2 oxo-2 tétrahydro-2H oxaazaphosphorine-1,3,2.

En remplaçant dans l'exemple 1, stade C, la [N--(chloro-2 éthyl) amido] -3 isopropyl-2 oxo-2 tétrahydro-2H oxaazaphosphorine-1,3,2 par la [N-(chloro-2 éthyl) amido] -3 méthoxy-2 oxo-2 tétrahydro-2H oxaazaphosphorine-1,3,2, on obtient après chromatographie rapide sur silice (cyclohexane-acétone; 70-30) la [N-(chloro-2 éthyl) N-nitrosoamido] -3 méthoxy-2 oxo-2 tétrahydro-2H oxaazaphosphorine-1,3,2 que l'on cristallise dans l'éther diisopropylique (Rendement 48%).

*Exemple 4*

[N-(chloro-2 éthyl) N-nitrosoamido] -3 [(méthyl-4 piperazinyl)-1] -2 oxo-2 tétrahydro-2H oxaazaphosphorine-1,3,2

*Stade A:* [(méthyl-4 pipérazinyl)-1-] -2 oxo-2 tétrahydro-2H oxaazaphosphorine-1,3,2.

En remplaçant dans l'exemple 1, stade A, le dichlorure de l'acide isopropyl phosphonique par le dichlorure du N-méthylpipérazide de l'acide phosphorique, on obtient la [(méthyl-3 pipérazinyl)-1] -2 oxo-2 tétrahydro-2H oxaazaphosphorine-1,3,2, qui est lavée à l'eau alcalinisée puis à l'eau distillée et utilisée telle quelle dans le stade suivant (Rendement 79%).

*Stade B:* [N-(chloro-2 éthyl) amido] -3 [(méthyl-4 pipérazinyl)-1] -2 oxo-2 tétrahydro-2H oxaazaphosphorine-1,3,2.

On chauffe à 35°C pendant 24 heures 15 grammes de [(méthyl-4 pipérazinyl)-1] -2 oxo-2 tétrahydro-2H oxaazaphosphorine-1,3,2 obtenue au stade précédent, dans 30 $cm^3$ de β-chloroéthyl isocyanate, puis évapore au maximum. On obtient 28 g de produit que l'on chromatographie sur colonne de silice ($CH_2Cl_2$-méthanol).

On isole 10,7 grammes de [N-(chloro-2 éthyl) amido] -3 [(méthyl-4 pipérazinyl)-1] -2 oxo-2 tétrahydro-2H oxaazaphosphorine-1,3,2 (Rendement 48%).

*Stade C:* [N-(chloro-2 éthyl) N-nitrosoamido] -3 [(méthyl-4 pipérazinyl)-1] -2 oxo-2 tetrahydro-2H oxaazaphosphorine-1,3,2.

En remplaçant dans l'exemple 1, stade C, la [N-(chloro-2 éthyl) amido] -3 isopropyl-2 oxo-2 tétrahydro-2H oxaazaphosphorine-1,3,2 par la [N-(chloro-2 éthyl) amido] -3 [(méthyl-4 pipérazinyl)-1] -2 oxo-2 tétrahydro-2H oxaazaphosphorine-1,3,2 mais en ajoutant le chlorure de nitrosyle à une température de −100°C et en agitant 30 minutes à −90°C, on obtient un résidu huileux qui est rapidement chromatographié sur silice (acétone-tétrahydrofuranne; 50-50) pour conduire à la [N-(chloro-2 éthyl) N-nitrosoamido] -3 [(méthyl-4 pipérazinyl)-1] -2 oxo-2 tétrahydro-2H oxaazaphosphorine-1,3,2 pure qui est ensuite dissoute dans un mélange acétate d'éthyle-acétone (1-1) et transformée, par adjonction de la quantité correspondante d'acide oxalique, en son oxalate.

*Exemples 5 à 13*

En appliquant le mode opératoire décrit dans l'exemple 1 et en utilisant le dichlorure de l'acide phosphorique ou de l'acide phosphonique correspondant, on prépare de la même façon les composés suivants:

5): [N-(chloro-2 éthyl) N-nitrosoamido] -3 méthyl-2 oxo-2 tétrahydro-2H oxaazaphosphorine-1,3,2.

6): [N-(chloro-2 éthyl) N-nitrosoamido] -3 (méthyl-1 propyl)-2 oxo-2 tétrahydro-2H oxaazaphosphorine-1,3,2.

7): [N-(chloro-2 éthyl) N-nitrosoamido] -3 cyclohexyl-2 oxo-2 tétrahydro-2H oxaazaphosphorine-1,3,2.

8): [N-(chloro-2 éthyl) N-nitrosoamido] -3 benzyl-2 oxo-2 tétrahydro-2H oxaazaphosphorine-1,3,2.

9): [N-(chloro-2 éthyl) N-nitrosoamido] -3 phénoxy-2 oxo-2 tétrahydro-2H oxaazaphosphorine-1,3,2.

10): [N-(chloro-2 éthyl) N-nitrosoamido] -3 [(chloro-4 phényl) méthyl] -2 oxo-2 tétrahydro-2H oxaazaphosphorine-1,3,2.

11): [N-(chloro-2 éthyl) N-nitrosoamido] -3 (chloro-4 phényl)-2 oxo-2 tétrahydro-2H oxaazaphosphorine-1,3,2.

12): [N-(chloro-2 éthyl) N-nitrosoamido] -3 (méthyl-4 phényl)-2 oxo-2 tétrahydro-2H oxaazaphosphorine-1,3,2.

13): [N-(chloro-2 éthyl) N-nitrosoamido] -3 (fluoro-4 phényl)-2 oxo-2 tétrahydro-2H oxaazaphosphorine-1,3,2.

*Exemples 14 à 19*

En utilisant le mode opératoire décrit dans l'exemple 4 à partir des dichlorures d'acides phosphoriques correspondants, on obtient de la même façon les composés suivants:

14): [N-(chloro-2 éthyl) N-nitrosoamido] -3 (pipéridinyl-1)-2 oxo-2 tétrahydro-2H oxaazaphosphorine-1,3,2.

15): [N-(chloro-2 éthyl) N-nitrosoamido] -3 (morpholinyl-4)-2 oxo-2 tétrahydro-2H oxaazaphosphorine-1,3,2.

16): [N-(chloro-2 éthyl) N-nitrosoamido] -3 [(méthoxy-2 phényl)-4 pipérazinyl-1] -2 oxo-2 tétrahydro-2H oxaazaphosphorine-1,3,2; chlorhydrate.

17): [N-(chloro-2 éthyl) N-nitrosoamido] -3 [(N-N bis chloro-2 éthyl) amino] -2 oxo-2 tétrahydro-2H oxaazaphosphorine-1,3,2.

18): [N-(chloro-2 éthyl) N-nitrosoamido] -3 [(cyclohexyl-4 pipérazinyl)-1] -2 oxo-2 tétrahydro-2H oxaazaphosphorine-1,3,2; oxalate.

19): [N-(chloro-2 éthyl) N-nitrosoamido] -3 [(butyl-4 pipérazinyl)-1] -2 oxo-2 tétrahydro-2H oxaazaphosphorine-1,3,2; oxalate.

*Etude pharmacologique des composés de l'invention*

Les composés de l'invention de formule générale I possèdent des propriétés pharmacologiques et thérapeutiques intéressantes. Ces dérivés ont une activité antitumorale élevée. Ils sont donc utiles dans le traitement de la maladie cancéreuse.

Ces composés sont testés par leur capacité à prolonger la survie de souris porteuses de cellules tumorales inoculées par voie intrapéritonéale, intramusculaire ou intracérébrale selon les protocoles recommandés par l'Institut National du Cancer (USA) (GERAN, R.I. et coll. Cancer Chemotherapy Reports, 1972, III, Vol. 3 N° 2, p. 1 à 87) et reconnus comme représentatifs de l'effet antitumoral en clinique humaine (DRISCOLL, J.S. Cancer Treatment Reports, 1984, Vol. 68 N° 1, p. 63 à 85). La puissance cytotoxique de ces composés à l'égard des cellules cancéreuses a été également mesurée par application du test de clonogénicité tel qu'il a été codifié par SALMON, S.E. et Von HOFF, D.D. (Semin. Oncol., 1981, Vol. 8 p. 3787).

Ces composés se sont révélés non seulement capables de ralentir la croissance des tumeurs greffées chez la souris, mais également de guérir des animaux rendus leucémiques. A titre d'exemple le composé de l'exemple 4 selon l'invention, dès la dose de 67 micromoles par kg en dose unique ou 22 micromoles en doses mutiples provoque, chez la souris des rémissions au delà de 60 jours après l'inoculation des

cellules cancéreuses. A cette même dose, il abolit la formation de métastases pulmonaires issues de l'injection d'un carcinome dans la patte.

Les composés selon l'invention présentent, de plus, un avantage considérable: l'absence de résistance croisée avec les nitrosourées. L'échec des chimiothérapies anticancéreuses résulte en effet du développement par la cellule cancereuse de parades qui la protègent de l'action destructrice des agents anticancéreux (CURT, G.A. et coll. 1984, Cancer Treatment Reports, 68, p. 87).

L'activité des composés selon l'invention a été testée sur des lignées de cellules sélectionnées pour leur résistance envers les nitrosourées [DCT Tumor Bank, Inventory of Transplantable Aminal and Human Tumors, NCI (USA), 30 juin 1981].

A titre d'exemple, le composé de l'exemple 4 selon l'invention prolonge de plus de 50% la survie de souris rendues leucémiques par l'inoculation intrapéritonéale de cent mille cellules de la lignée L1210/BCNU alors que le BCNU est incapable de prolonger significativement leur survie (voir tableau ci-après).

ACTIVITE CONTRE LE DEVELOPPEMENT DE LA LEUCEMIE MURINE L1210 RESISTANTE AU BCNU

| Composé | Nombre d'animaux par lot | Dose unitaire par traitement (µ mole/kg) | Nombre de traitements | Jours de(s) traitement(s) (jour) (*) | Temps moyen de survie (jour) | % Augment. temps survie/ contrôles (**) |
|---|---|---|---|---|---|---|
| Contrôle (***) | 30 | – | – | – | 9,3 | 0 |
| BCNU | 6 | 138 | 1 | 1 | 11,2 | 20 |
| Composé N° 4 | 6 | 84 | 1 | 1 | 14 | 51 |
| – | 6 | 42 | 3 | 1,5,9 | 14,3 | 54 |
| – | 6 | 21 | 9 | 1 à 9 | 14,8 | 59 |

(*) Le jour 0 de l'expérience est le jour de l'inoculation intrapéritonéale de 100 000 cellules L1210/BCNU.

(**) La valeur de 25% est requise pour attester d'une activité antitumorale significative sur ce modèle.

(***) Lot contrôle = animaux inoculés avec la tumeur, traités seulement par le véhicule d'administration (HPC 0,2%, NaCl 0,15 M).

In vitro, des cellules cancéreuses traitées par des concentrations aussi faibles que 0,5 nanomole par millilitre du composé de l'exemple 4 selon l'invention sont incapables de former des colonies de cellules malignes, alors qu'il faut plus de 14 nanomoles par millilitre de N-N'-bis (2-chloroéthyl)-N-nitrosurée («BCNU») composé de la même classe thérapeutique le plus utilisé en clinique. De plus le composé selon l'invention maintenu en solution pendant plus de 3 heures à 37°C dans le milieu de culture complet, ne perd aucune de ses propriétés cytotoxiques tandis que, pendant le même temps la puissance du BCNU décroît considérablement.

Les composés selon l'invention apparaissent peu toxiques. A titre d'exemple, trois injections par voie intrapéritonéale du composé de l'exemple 4 selon l'invention, chacune à 4 jours d'intervalle, chez la souris B6D2F1 à la dose de 67 micromoles par kg, ne diminuent jamais la densité des leucocytes du sang périphérique en dessous de 70% de la valeur initiale. A une dose équipotente le BCNU fait chuter la formule leucocytaire à une valeur inférieure de la moitié de la valeur normale. Les lignées érythrocytaires et thrombopoiétiques ne sont pas perturbées significativement par le traitement par les produits selon l'invention. On peut corréler cette excellente propriété à l'incapacité du composé selon l'invention, de carbamoyler des molécules de lysine avec lesquelles il a été mélangé pendant 6 heures à 37°C.

En effet, il a été soutenu (KANN, H.E. 1981, in: NITROSOUREAS: Current Status and New Developments Prestayko, A.W. et coll. Editeurs, Acad. Press. p. 96) que le pouvoir carbamoylant de certaines nitrosourées expose les tissus normaux à des effets toxiques indésirables. Les composés selon l'invention présentent donc l'étonnante qualité d'être très actifs sur les cellules cancéreuses et, par ailleurs, de ne pouvoir altérer des composants essentiels à la survie des cellules normales.

Les composés selon l'invention sont utiles chez l'homme, et les animaux en cas de leucémies, myélomes, carcinomes, sarcomes, mélanomes, épithéliomes, gliomes, tératomes et plus généralement des cancers de toutes localisations.

L'invention s'étend aussi aux compositions pharmaceutiques renfermant comme principe actif au moins un composé de formule générale I, ou éventuellement un de ses sels d'addition à un acide pharmaceutiquement acceptable, seul ou associé à un ou plusieurs excipients inertes non-toxiques convenant pour l'usage pharmaceutique. Les compositions pharmaceutiques ainsi obtenues sont avantageusement présentées sous des formes galéniques diverses, telles que par exemple des comprimés, dragées, gélules, capsules, cachets, suppositoires, solutions injectables ou buvables, ou des préparations appropriées à une administration sublinguale.

La posologie utile peut varier largement en fonction de l'âge et du poids du patient, de la nature de la localisation et de la sévérité de l'affection cancéreuse. La voie d'administration préférée est la voie orale, mais les voies rectale ou parentérale sont également appropriées. D'une manière générale, le dosage unitaire s'échelonnera de préférence entre 5 et 100 mg.

*Exemple de formulation*

| | |
|---|---|
| [N-(chloro-2 éthyl) N-nitrosoamido] -3 [(méthyl-4) pipérazinyl)-1] -2 oxo-2 tétrahydro-2H-oxaazaphosphorine-1,3,2 oxalate | 20 mg |
| Talc | 5 mg |
| Lactose | 25 mg |

pour une gélule.

TABLEAU I

| Composés N° | R | Rdt % | F°C | IR ($cm^{-1}$) | RMN |
|---|---|---|---|---|---|
| 1 | $-CH(CH_3)_2$ | 81 | 84 | ν C=O : 1690 | 1,05 ppm, 3H, d de d, ($J_{PH}$ = 2 Hz; $J_{HH}$ = 6,3 Hz; 1,4 ppm, 3H, d, $J_{HH}$ = 6,3 Hz, 1,9 à 2,8 ppm, 3H, m; 3,2 à 3,8, 3H, m; 4 à 4,7, 5H, m |
| 2 | $-C_6H_5$ | 62 | 74 | ν C=O : 1700 | 1,8 à 3 ppm, 2H, m; 3,3 à 3,9 ppm, 3H, m; 3,9 à 4,9 ppm, 5H, m; 7,3 à 8,1 ppm, 5H, m |
| 3 | $-OCH_3$ | 48 | 65 | ν C=O : 1710 | 1,7 à 2,8 ppm, 2H, m; 3,2 à 4,7 ppm, 11H, m; 3,8 ppm, 3H, d, $J_{PH}$ = 11,6 $H_3$ |
| 4 | 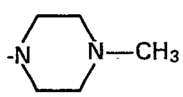 oxalate | 45 | 110 | ν$NH^+$ et OH:2000 à 3700<br>ν C = O : 1700 | 1,8 à 2,3 ppm, 2H, m; 2,7 ppm, 3H, s, 2,8 à 4,6 ppm, 16H, m; 10,6 ppm, 2H, échangeables |
| 5 | $-CH_3$ | 51 | 105 | ν C = O : 1690 | 1,6 à 1,9 ppm, 3H, d, ($J_{PH}$ = 18 Hz, 2 à 2,5 ppm, 2H, m; 3,2 à 3,8 ppm, 3H, m; 4 à 4,8 ppm, 5H, m |
| 6 | $-CH(CH_3)C_2H_5$ | 43 | huile | ν C = O : 1705 | 0,9 à 1,1 ppm, 3H, m; 1,4 ppm, 3H, d, 1,5 à 2,8 ppm, 5H, m; 3,2 à 4 ppm, 3H, m; 4 à 4,8 ppm, 5H, m |
| 7 | cyclohexyl | 47 | huile | ν C = O : 1705 | 1 à 2,7 ppm, 13H, m; 3,2 à 3,8 ppm, 3H, m; 4 à 4,7 ppm, 5H, m |
| 8 | $-CH_2-C_6H_5$ | 71 | 99 | ν C = O : 1705 | 1,7 à 3,1 ppm, 3H, m; 3,35 ppm, 2H, d; 3,5 à 4,7 ppm, 7H, m; 7,25 ppm, 5H, s |
| 9 | $-O-C_6H_5$ | 60 | huile | ν C = O : 1715<br>ν C = O : 1590 | 2,1 ppm, 2H, m; 3,2 à 4 ppm, 3H, m; 4 à 5 ppm, 5H, m; 7 à 7,25 ppm, 5H, m |
| 10 | 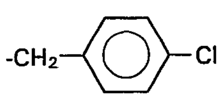 | 73 | 90 | ν C = O : 1690 | 1,8 à 3,1 ppm, 3H, m; 3,4 ppm, 2H, d; 3,6 ppm, 2H, m; 3,8 à 4,8 ppm, 5H, m; 7,4 ppm, 4H, s |
| 11 | 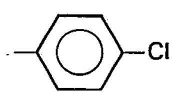 | 33 | 90 | ν C = O : 1710 | 2 à 3 ppm, 2H, m; 3,3 à 4,8 ppm, 8H, m; 7,3 à 8 ppm, 4H, m |
| 12 | 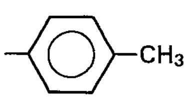 | 30 | 80 | νC = O : 1700 | 2 à 2,7 ppm, 3H, m; 2,4 ppm, 3H, s; 3,3 à 5 ppm, 8H, m; 7,2 à 8 ppm, 4H, m |
| 13 | 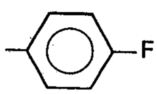 | 80 | 103 | ν C = O :1700 | 1,8 à 2,6 ppm, 2H, m; 3,3 à 4,8 ppm, 8H, m; 7 à 8,2 ppm, 4H, m |

TABLEAU I (suite)

| Composés N° | R | Rdt % | F°C | IR ($cm^{-1}$) | RMN |
|---|---|---|---|---|---|
| 14 | 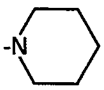 | 31 | 92 | ν C = O : 1695 | 1,3 à 2,5 ppm, 8H, m; 2,8 à 3,3 ppm, 4H, m; 3,3 à 3,8 ppm, 3H, m; 3,9 à 4,7 ppm, 5H, m |
| 15 | 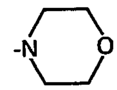 | 45 | 105 | ν C = O : 1710 | 1,9 à 2,5 ppm, 2H, m; 3 à 4 ppm, 10H, m; 3,9 à 4,7 ppm, 6H, m |
| 16 | 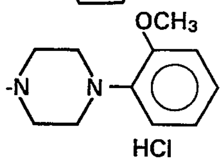 | 38 | 165 | ν C = O : 1715<br>νNH: 1900-2800 | 1,9 à 2,3 ppm, 2H, m; 3,3 à 4,7 ppm, 19H, m; 6,9 à 7,8 ppm, 4H, m; 6,5 ppm, proton échangeable |
| 17 | $-N(CH_2-CH_2Cl)_2$ | 65 | 79 | ν C = O : 1690 | 1,8 à 2,5 ppm, 2H, m; 2,5 à 3,9 ppm, 11H, m; 3,9 à 4,5 ppm, 3H, m |
| 18 | 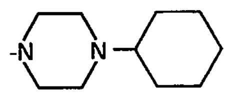 | 64 | 121 | ν C = O : 1720 | 0,8 à 2,3 ppm, 12H, m; 2,7 à 3,8 ppm, 12H, m; 3,8 à 4,7 ppm, 5H, m; 5,1 ppm, 2H, m, (échangeables) |
| 19 | 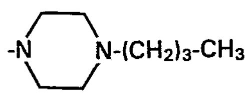 | 50 | 92 | ν C = O : 1650<br>et 1700 | 0,7 à 2,4 ppm, 9H, m; 2,7 à 4,6 ppm, 18H, m; 7,9 ppm, 2H m, (échangeables) |

**Revendications pour les Etats constractants:**
BE, CH/LI, DE, FR, GB, IT, LU, NL, SE

1. Dérivés de l'oxaazaphosphorine répondant à la formule générale I:

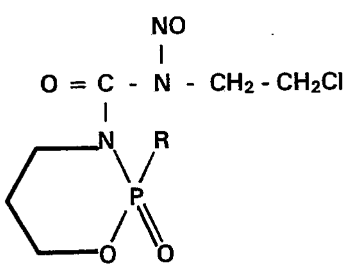

I

dans laquelle R représente:

- un groupe alkyle en chaîne droite ou ramifiée comportant de 1 à 6 atomes de carbone,
- un groupe cycloalkyle comportant de 3 à 7 atomes de carbone,
- un groupe phényle pouvant être substitué par un radical alkyle ou alcoxy comportant de 1 à 4 atomes de carbone ou par un atome d'halogène,
- un groupe phénylalkyle comportant de 7 à 10 atomes de carbone dans lequel le noyau phényle peut-être éventuellement substitué par un atome d'halogène ou un radical alkyle ou alcoxy de 1 à 4 atomes de carbone,
- un groupe alcoxy de 1 à 4 atomes de carbone en chaîne droite ou ramifiée,
- un groupe phénoxy ou thiophénoxy,
- un groupe mono- ou di-alkylamino comportant de 1 à 8 atomes de carbone pouvant être éventuellement substitué par un ou deux atomes d'halogène, ou
- un groupe pyrrolidinyle-1, morpholinyle-4, pipéridinyle-1 ou un groupe pipérazinyle-1 éventuellement substitué en position 4 par un reste alkyle de 1 à 4 atomes de carbone en chaîne droite ou ramifiée, par un groupe cycloalkyle de 5 à 7 atomes de carbone, ou par un groupe phényle ou phénylalkyle de 7 à 10 atomes de carbone dans lesquels le noyau phényle peut être substitué par un atome d'halogène ou un radical alkyle ou alcoxy comportant de 1 à 4 atomes de carbone en chaîne droite ou ramifiée,

ainsi que, lorsque R représente un groupe diaminé, leurs sels d'addition à des acides pharmaceutiquement acceptables.

2. Composés selon la revendication 1 dans laquelle R représente un groupe aminé et en particulier un radical pyrrolidinyle-1, pipéridinyle-1, morpholinyle-4 ou pipérazinyle-1 éventuellement substitué en position 4 par un goupe alkyle de 1 à 4 atomes de carbone en chaîne droite ou ramifiée, par un groupe cycloalkyle de 5 à 7 atomes de carbone, ou par un groupe phényle ou phénylalkyle de 7 à 10 atomes de carbone dans lesquels le noyau phényle peut être substitué par un atome d'halogène ou un radical alkyle ou alcoxy comportant de 1 à 4 atomes de carbone en chaîne droite ou ramifiée.

3. La [N-(chloro-2 éthyl) N-nitrosoamido] -3 (pipéridinyl-1) -2 oxo-2 tétrahydro-2H oxaazaphosphorine-1,3,2.

4. La [N-(chloro-2 éthyl) N-nitrosoamido] -3 (morpholinyl-4) -2 oxo-2 tétrahydro-2H oxaazaphosphorine-1,3,2.

5. La [N-(chloro-2 éthyl) N-nitrosoamido] -3 [(méthyl-4 pipérazinyl)-1] -2 oxo-2 tétrahydro-2H oxaazaphosphorine-1,3,2, et ses sels d'addition à un acide pharmaceutiquement acceptable.

6. La [N-(chloro-2 éthyl) N-nitrosoamido] -3 [(méthoxy-2 phényl) -4 pipérazinyl-1] -2 oxo-2 tétrahydro-2H oxaazaphosphorine-1,3,2, et ses sels d'addition à un acide pharmaceutiquement acceptable.

7. Procédé de préparation des composés selon la revendication 1, caractérisé en ce que l'on condense un dichlorure d'acide phosphonique ou d'acide phosphorique de formule II:

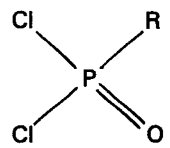

II

dans laquelle R a la signification définie dans la revendication 1,

avec l'amino-3 propanol-1 dans un solvant inerte et en présence d'un accepteur d'acide à une température comprise entre −30 et 0°C, en un composé de formule III:

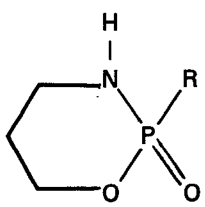

III

dans laquelle R a la signification définie dans la revendication 1,

que l'on condense avec un excès de β-chloro-éthyl isocyanate de formule IV:

$$Cl\text{-}CH_2\text{-}CH_2\text{-}N=C=O \qquad IV$$

soit directement lorsque R représente un groupe aminé, soit quand R est un groupe non-aminé, après activation préalable de l'atome d'azote du cycle oxaazaphosphorine-1,3,2 par le butyllithium pour obtenir en un composé de formule V:

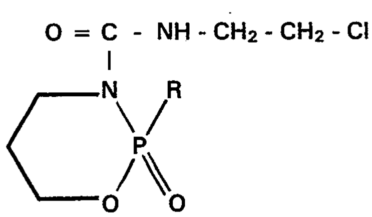

V

dans laquelle R a la signification définie dans la revendication 1,

qui est ensuite nitrosé en milieu acide ou basique par action d'agents nitrosants en un composé de formule I,

qui peut-être ensuite transformée, lorsque R est un groupe diaminé, en son sel d'addition à un acide pharmaceutiquement acceptable.

8. Procédé selon la revendication 7 caractérisé en ce que la nitrosation d'un dérivé de formule V est réalisée après activation préalable de l'atome d'azote de l'urée.

9. Procédé selon les revendications 7 et 8 caractérisé en ce que l'activation de l'atome d'azote préalable à la nitrosation d'un dérivé de formule V est réalisée par le butyllithium dans le tétrahydrofuranne à une température inférieure à −60°C.

10. Procédé selon l'une des revendications 8 et 9 caractérisé en ce que les dérivés de formule V sont nitrosés par le chlorure de nitrosyle, le nitrite de sodium ou le tétroxyde d'azote dans un solvant acide ou basique.

11. Compositions pharmaceutiques contenant comme principe actif au moins un composé selon l'une quelconque des revendications 1 à 6, seul ou en association avec un ou plusieurs excipients inertes non-toxiques.

12. Compositions pharmaceutiques contenant comme principe actif la [N-(chloro-2 éthyl) N-nitrosoamido] -3 [(méthyl-4 pipérazinyl)-1] -2 oxo-2 tétrahydro-2H oxaazaphosphorine-1,3,2, ou l'un de ses sels d'addition à un acide pharmaceutiquement acceptable, seul ou en association avec un ou plusieurs excipients inertes non-toxiques.

**Revendications pour l'Etat contractant:** AT

1. Procédé de préparation de nouveaux dérivés de l'oxaazaphosphorine répondant à la formule générale I:

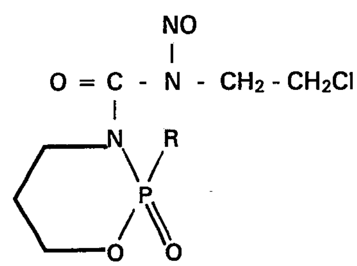

I

dans laquelle R représente:

– un groupe alkyle en chaîne droite ou ramifiée comportant de 1 à 6 atomes de carbone,

– un groupe cycloalkyle comportant de 3 à 7 atomes de carbone,

– un groupe phényle pouvant être substitué par un radical alkyle ou alcoxy comportant de 1 à 4 atomes de carbone ou par un atome d'halogène,

– un groupe phénylalkyle comportant de 7 à 10 atomes de carbone dans lequel le noyau phényle peut-être éventuellement substitué par un atome d'halogène ou un radical alkyle ou alcoxy de 1 à 4 atomes de carbone,

– un groupe alcoxy de 1 à 4 atomes de carbone en chaîne droite ou ramifiée,

– un groupe phénoxy ou thiophénoxy,

– un groupe mono- ou di-alkylamino comportant de 1 à 8 atomes de carbone pouvant être éventuellement substitué par un ou deux atomes d'halogène, ou

– un groupe pyrrolidinyle-1, morpholinyle-4, pipéridinyle-1 ou un groupe pipérazinyle-1 éventuellement substitué en position 4 par un reste alkyle de 1 à 4 atomes de carbone en chaîne droite ou ramifiée, par un groupe cycloalkyle de 5 à 7 atomes de carbone, ou par un groupe phényle ou phénylalkyle de 7 à 10 atomes de carbone dans lesquels le noyau phényle peut être substitué par un atome d'halogène ou un radical alkyle ou alcoxy comportant de 1 à 4 atomes de carbone en chaîne droite ou ramifiée,

ainsi que, lorsque R représente un groupe diaminé, leurs sels d'addition à des acides pharmaceutiquement acceptables,

caractérisé en ce que l'on condense un dichlorure d'acide phosphonique ou d'acide phosphorique de formule II:

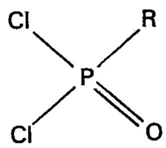

II

dans laquelle R a la signification définie dans la formule I,

avec l'amino-3 propanol-1 dans un solvant inerte et en présence d'un accepteur d'acide à une température comprise entre −30 et 0 °C, en un composé de formule III:

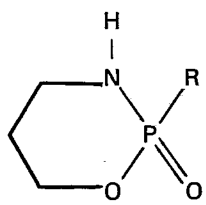

III

dans laquelle R a la signification définie dans la formule I,

que l'on condense avec un excès de β-chloro-éthyl isocyanate de formule IV:

$$Cl\text{-}CH_2\text{-}CH_2\text{-}N=C=O \qquad IV$$

soit directement lorsque R représente un groupe aminé, soit quand R est un groupe non-aminé, après activation préalable de l'atome d'azote du cycle oxazaphosphorine-1,3,2 par le butyllithium pour obtenir en un composé de formule V:

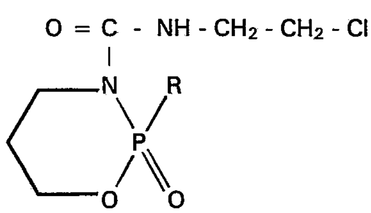

V

dans laquelle R a la signification définie dans la formule I,

qui est ensuite nitrosé en milieu acide ou basique par action d'agents nitrosants en un composé de formule I,

qui peut-être ensuite transformé, lorsque R est un groupe diaminé, en son sel d'addition à un acide pharmaceutiquement acceptable.

2. Procédé selon la revendication 1 caractérisé en ce que la nitrosation d'un dérivé de formule V est réalisée après activation préalable de l'atome d'azote de l'urée.

3. Procédé selon les revendications 1 et 2 caractérisé en ce que l'activation de l'atome d'azote préalable à la nitrosation d'un dérivé de formule V est réalisée par le butyllithium dans le tétrahydrofuranne à une température inférieure à −60 °C.

4. Procédé selon l'une des revendications 1 à 3 caractérisé en ce que les dérivés de formule V sont nitrosés par le chlorure de nitrosyle, le nitrite de sodium ou le tétroxyde d'azote dans un solvant acide ou basique.

**Patentansprüche für die Vertragsstaten:**
BE, CH/LI, DE, FR, GB, IT, LU, NL, SE

1. Oxaazaphosphorinderivate der allgemeinen Formel I

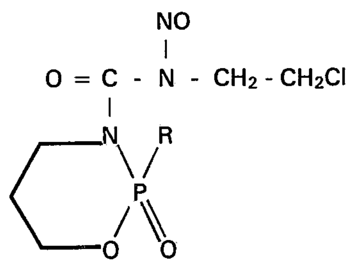

I

in der R

– eine Alkylgruppe mit gerader oder verzweigter Kette und 1 bis 6 Kohlenstoffatomen,

– eine Cycloalkylgruppe mit 3 bis 7 Kohlenstoffatomen,

– eine Phenylgruppe, die durch eine Alkyl- oder Alkoxygruppe mit 1 bis 4 Kohlenstoffatomen oder ein Halogenatom substituiert sein kann,

– eine Phenylalkylgruppe mit 7 bis 10 Kohlenstoffatomen, deren Phenylkern gegebenenfalls durch ein Halogenatom oder eine Alkyl- oder Alkoxygruppe mit 1 bis 4 Kohlenstoffatomen substituiert sein kann,

– eine Alkoxygruppe mit 1 bis 4 Kohlenstoffatomen mit gerader oder verzweigter Kette,

– eine Phenoxygruppe oder eine Thiophenoxygruppe,

– eine Mono- oder Di-Alkylaminogruppe mit 1 bis 8 Kohlenstoffatomen, die gegebenenfalls durch ein oder zwei Halogenatome substituiert sein kann, oder

– eine Pyrrolidin-1-yl, Morpholin-4-yl, Piperidin-1-yl, oder Piparazin-1-yl-Gruppe, die gegebenenfalls in der 4-Stellung durch eine Alkylgruppe mit 1 bis 4 Kohlenstoffatomen mit gerader oder verzweigter Kette, durch eine Cycloalkylgruppe mit 5 bis 7 Kohlenstoffatomen oder durch eine Phenyl- oder Phenylalkylgruppe mit 7 bis 10 Kohlenstoffatomen, deren Phenylrest gegebenenfalls durch ein Halogenatom

oder eine Alkyl- oder Alkoxygruppe mit 1 bis 4 Kohlenstoffatomen mit gerader oder verzweigter Kette substituiert ist, substituiert sein kann, bedeutet,

sowie die Additionssalze der Verbindungen, worin R eine diaminierte Gruppe darstellt, mit pharmazeutisch annehmbaren Säuren.

2. Verbindungen nach Anspruch 1, dadurch gekennzeichnet, dass R eine aminierte Gruppe darstellt, insbesondere eine Pyrrolidin-1-yl-, Piperidin-1-yl-, Morpholin-4-yl- oder Piperazin-1-yl-Gruppe, die gegebenenfalls in der 4-Stellung durch eine Alkylgruppe mit 1 bis 4 Kohlenstoffatomen in gerader oder verzweigter Kette, eine Cycloalkylgruppe mit 5 bis 7 Kohlenstoffatomen oder eine Phenyl- oder Phenylalkylgruppe mit 7 bis 10 Kohlenstoffatomen, deren Phenylkern gegebenenfalls durch ein Halogenatom oder eine Alkyl- oder Alkoxygruppe mit 1 bis 4 Kohlenstoffatomen in gerader oder verzweigter Kette substituiert ist, substituiert sein kann, bedeutet.

3. 3-[N-(2-Chlor-ethyl)-N-nitrosoamido]-2-(piperidin-1-yl)-2-oxo-2H-tetrahydro-1,3,2-oxaazaphosphorin.

4. 3-[N-(2-Chlor-ethyl)-N-nitrosoamido]-2-(morpholin-4-yl)-2-oxo-2H-tetrahydro-1,3,2-oxaazaphosphorin.

5. 3-[N-(2-Chlor-ethyl)-N-nitrosoamido]-2-[1-(4-methyl-piperazinyl)]-2-oxo-2H-tetrahydro-1,3,2-oxaazaphosphorin und dessen Additionssalze mit einer pharmazeutisch annehmbaren Säure.

6. 3-[N-(2-Chlor-ethyl)-N-nitrosoamido]-2-[4-(2-methoxy-phenyl)-piperazin-1-yl]-2-oxo-2H-tetrahydro-1,3,2-oxaazaphosphorin und dessen Additionssalze mit einer pharmazeutisch annehmbaren Säure.

7. Verfahren zur Herstellung der Verbindungen nach Anspruch 1, dadurch gekennzeichnet, dass man ein Dichlorid der Phosphonsäure oder der Phosphorsäure der Formel II

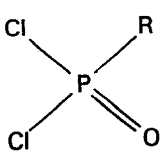

II

worin R die in Anspruch 1 angegebenen Bedeutungen besitzt,

mit 3-Amino-propanol-1 in einem inerten Lösungsmittel und in Gegenwart eines Säureakzeptors bei einer Temperatur zwischen −30 und 0°C zu einer Verbindung der Formel III

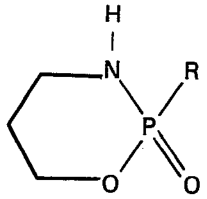

III

worin R die in Anspruch 1 angegebenen Bedeutungen besitzt, kondensiert,

die man dann mit einem Überschuss von β-Chlorethylisocyanat der Formel IV

$$Cl\text{-}CH_2\text{-}CH_2\text{-}N=C=O \qquad IV$$

entweder direkt, wenn R eine aminierte Gruppe darstellt, oder nach einer vorausgehenden Aktivierung des Stickstoffatoms des 1,3,2-Oxaazaphosphorinringes mit Butyllithium wenn R eine nichtaminierte Gruppe darstellt, kondensiert, unter Bildung einer Verbindung der Formel V

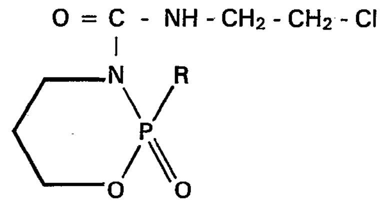

V

worin R die in Anspruch 1 angegebenen Bedeutungen besitzt, die anschliessend in saurem oder basischem Medium mit einem Nitrosierungsmittel zu einer Verbindung der Formel I nitrosiert wird, die anschliessend, wenn R eine diaminierte Gruppe darstellt, in das Additionssalz mit einer pharmazeutisch annehmbaren Säure umgewandelt wird.

8. Verfahren nach Anspruch 7, dadurch gekennzeichnet, dass die Nitrosierung des Derivats der Formel V nach vorausgehender Aktivierung des Stickstoffatoms von Harnstoff durchgeführt wird.

9. Verfahren nach den Ansprüchen 7 und 8, dadurch gekennzeichnet, dass die der Nitrosierung vorausgehende Aktivierung des Stickstoffatoms des Derivats der Formel V mit Butyllithium in Tetrahydrofuran bei einer Temperatur von weniger als −60°C durchgeführt wird.

10. Verfahren nach einem der Ansprüche 8 und 9, dadurch gekennzeichnet, dass die Derivate der Formel V mit Nitrosylchlorid, Natriumnitrid oder Stickstofftetroxid in einem sauren oder basischen Medium nitrosiert werden.

11. Pharmazeutische Zubereitungen enthaltend als Wirkstoff mindestens eine Verbindung nach einem der Ansprüche 1 bis 6 allein oder in Kombination mit einem oder mehreren inerten, nichttoxischen Trägern.

12. Pharmazeutische Zubereitungen enthalternd als Wirkstoff 3-[N-(2-Chlor-ethyl)-N-nitrosoamido]-2-[1-(4-methyl-piperazinyl)]-2-oxo-2H-tetrahydro-1,3,2-oxaazaphosphorin oder eines seiner Additionssalze mit einer pharmazeutisch annehmbaren Säure allein oder in Kombination mit einem oder mehreren inerten, nichttoxischen Trägern.

**Patentansprüche für den Vertragsstaat:** AT

1. Verfahren zur Herstellung von neuen Oxaazaphosphorinderivaten der allgemeinen Formel I

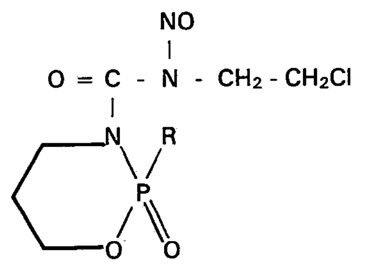

I

in der R

- eine Alkylgruppe mit gerader oder verzweigter Kette und 1 bis 6 Kohlenstoffatomen,
- eine Cycloalkylgruppe mit 3 bis 7 Kohlenstoffatomen,
- eine Phenylgruppe, die durch eine Alkyl- oder Alkoxygruppe mit 1 bis 4 Kohlenstoffatomen oder ein Halogenatom substituiert sein kann,
- eine Phenylalkylgruppe mit 7 bis 10 Kohlenstoffatomen, deren Phenylkern gegebenenfalls durch ein Halogenatom oder eine Alkyl- oder Alkoxygruppe mit 1 bis 4 Kohlenstoffatomen substituiert sein kann,
- eine Alkoxygruppe mit 1 bis 4 Kohlenstoffatomen mit gerader oder verzweigter Kette,
- eine Phenoxygruppe oder eine Thiophenoxygruppe,
- eine Mono- oder Di-Alkylaminogruppe mit 1 bis 8 Kohlenstoffatomen, die gegebenenfalls durch ein oder zwei Halogenatome substituiert sein kann, oder
- eine Pyrrolidin-1-yl, Morpholin-4-yl, Piperidin-1-yl, oder Piparazin-1-yl-Gruppe, die gegebenenfalls in der 4-Stellung durch eine Alkylgruppe mit 1 bis 4 Kohlenstoffatomen mit gerader oder verzweigter Kette, durch eine Cycloalkylgruppe mit 5 bis 7 Kohlenstoffatomen oder durch eine Phenyl- oder Phenylalkylgruppe mit 7 bis 10 Kohlenstoffatomen, deren Phenylrest gegebenenfalls durch ein Halogenatom oder eine Alkyl- oder Alkoxygruppe mit 1 bis 4 Kohlenstoffatomen mit gerader oder verzweigter Kette substituiert ist, substituiert sein kann, bedeutet,

sowie die Additionssalze der Verbindungen, worin R eine diaminierte Gruppe darstellt, mit pharmazeutisch annehmbaren Säuren, dadurch gekennzeichnet, dass man ein Dichlorid der Phosphonsäure oder der Phosphorsäure der Formel II

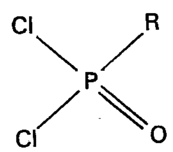

II

worin R die für die Formel I angegebenen Bedeutungen besitzt,

mit 3-Amino-propanol-1 in einem inerten Lösungsmittel und in Gegenwart eines Säureakzeptors bei einer Temperatur zwischen −30 und 0°C zu einer Verbindung der Formel III

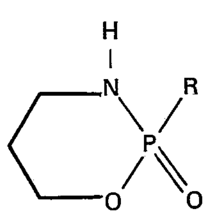

III

worin R die für die Formel I angegebenen Bedeutungen besitzt, kondensiert,

die man dann mit einem Überschuss von β-Chlorethylisocyanat der Formel IV

$Cl\text{-}CH_2\text{-}CH_2\text{-}N=C=O$ IV

entweder direkt, wenn R eine aminierte Gruppe darstellt oder nach einer vorausgehenden Aktivierung des Stickstoffatoms des 1,3,2-Oxaazaphosphorinringes mit Butyllithium wenn R eine nichtaminierte Gruppe darstellt, unter Bildung einer Verbindung der Formel V

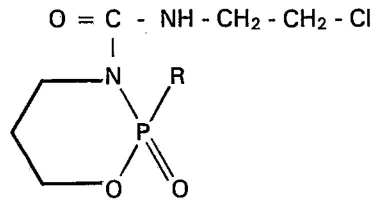

V

worin R die für die Formel I angegebenen Bedeutungen besitzt, die anschliessend in saurem oder basischem Medium mit einem Nitrosierungsmittel zu einer Verbindung der Formel I nitrosiert wird, die anschliessend, wenn R eine diaminierte Gruppe darstellt, in das Additionssalz mit einer pharmazeutisch annehmbaren Säure umgewandelt wird.

2. Verfahren nach Anspruch 1, dadurch gekennzeichnet, dass die Nitrosierung des Derivats der Formel V nach vorausgehender Aktivierung des Stickstoffatoms von Harnstoff durchgeführt wird.

3. Verfahren nach den Ansprüchen 1 und 2, dadurch gekennzeichnet, dass die der Nitrosierung vorausgehende Aktivierung des Stickstoffatoms des Derivats der Formel V mit Butyllithium in Tetrahydrofuran bei einer Temperatur von weniger als −60°C durchgeführt wird.

4. Verfahren nach einem der Ansprüche 2 und 3, dadurch gekennzeichnet, dass die Derivate der Formel V mit Nitrosylchlorid, Natriumnitrid oder Stickstofftetroxid in einem sauren oder basischen Medium nitrosiert werden.

**Claims for the Contracting States:**
BE, CH/LI, DE, FR, GB, IT, LU, NL, SE

1. Oxaazaphosphorine derivatives corresponding to the general formula I

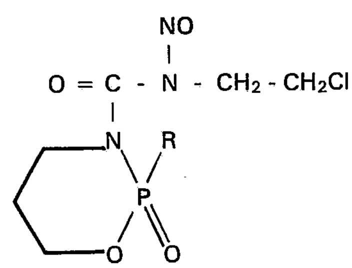

I

in which R represents:

- a straight-chain or branched-chain alkyl group containing from 1 to 6 carbon atoms,
- a cycloalkyl group containing from 3 to 7 carbon atoms,
- a phenyl group that may be substituted by an alkyl or alkoxy radical containing from 1 to 4 carbon atoms or by a halogen atom,
- a phenylalkyl group that contains from 7 to 10 carbon atoms and in which the phenyl ring may optionally be substituted by a halogen atom or an alkyl or alkoxy radical containing from 1 to 4 carbon atoms,

- a straight-chain or branched-chain alkoxy group containing from 1 to 4 carbon atoms,

- a phenoxy or thiophenoxy group,

- a mono- or di-alkylamino group that contains from 1 to 8 carbon atoms and that may optionally be substituted by one or two halogen atoms, or

- a pyrrolidin-1-yl, morpholin-4-yl or piperid-1-yl group, or a piperazin-1-yl group that is optionally substituted in the 4-position by a straight-chain or branched-chain alkyl radical containing from 1 to 4 carbon atoms, by a cycloalkyl group containing from 5 to 7 carbon atoms, or by a phenyl or phenylalkyl group that contains from 7 to 10 carbon atoms and in which the phenyl ring may be substituted by a halogen atom or a straight-chain or branched-chain alkyl or alkoxy radical containing from 1 to 4 carbon atoms,

and also, when R represents a diamino group, their pharmaceutically acceptable acid addition salts.

2. Compounds according to claim 1 in which R represents an amino group and, especially, a radical pyrrolidin-1-yl, piperid-1-yl, morpholin-4-yl or piperazin-1-yl that is optionally substituted in the 4-position by a straight-chain or branched-chain alkyl group containing from 1 to 4 carbon atoms, by a cycloalkyl group containing from 5 to 7 carbon atoms, or by a phenyl or phenylalkyl group that contains from 7 to 10 carbon atoms and in which the phenyl ring may be substituted by a halogen atom or a straight-chain or branched-chain alkyl or alkoxy radical containing from 1 to 4 carbon atoms.

3. 3-[N-(2-chloroethyl)-N-nitrosoamido]-2-(piperid-1-yl)-2-oxo-2H-tetrahydro-1,3,2-oxaazaphosphorine.

4. 3-[N-(2-chloroethyl)-N-nitrosoamido]-2-(morpholin-4-yl)-2-oxo-2H-tetrahydro-1,3,2-oxaaza-phosphorine.

5. 3-[N-(2-chloroethyl)-N-nitrosoamido]-2-[1-(4--methylpiperazinyl)]-2-oxo-2H-tetrahydro-1,3,2--oxaazaphosphorine and its pharmaceutically acceptable acid addition salts.

6. 3-[N-(2-chloroethyl)-N-nitrosoamido]-2-[4-(2--methoxyphenyl)-piperazin-1-yl]-2-oxo-2H-tetrahydro-1,3,2-oxaazaphosphorine and its pharmaceutically acceptable acid addition salts.

7. Process for the preparation of the compounds according to claim 1, characterised in that a phosphonic or phosphoric acid dichloride of the formula II

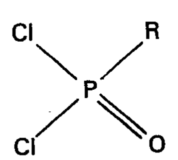

II

in which R has the meaning defined in claim 1,

is condensed with 3-aminopropan-1-ol in an inert solvent and in the presence of an acid acceptor at a temperature between −30 and 0°C to form a compound of the formula III

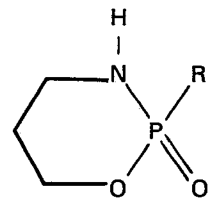

III

in which R has the meaning defined in claim 1,

and wich is condensed with an excess of β-chloroethyl isocyanate of the formula IV

$$Cl\text{-}CH_2\text{-}CH_2\text{-}N=C=O \quad IV$$

either directly, when R represents an amino group, or, when R is not an amino group, after previously activating the nitrogen atom of the 1,3,2-oxaazaphosphorine ring by means of butyllithium, to obtain a compound of the formula V

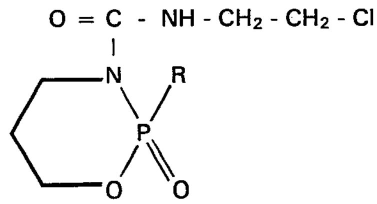

V

in which R has the meaning defined in claim 1,

and which is then nitrosated in an acidic or basic medium by the action of nitrosating agents, to form a compound of the formula I,

which, when R is a diamino group, can then be converted into its pharmaceutically acceptable acid addition salt.

8. Process according to claim 7, characterised in that the nitrosation of a derivative of the formula V is carried out after previously activating the nitrogen atom of the urea.

9. Process according to claims 7 and 8, characterised in that the activation of the nitrogen atom before nitrosating a derivative of the formula V is carried out by means of butyllithium in tetrahydrofuran at a temperature of less than −60°C.

10. Process according to either of claims 8 and 9, characterised in that the derivatives of the formula V are nitrosated by nitrosyl chloride, sodium nitrite or nitrogen textroxide in an acidic or basic solvent.

11. Pharmaceutical compositions containing as active ingredient at least one compound according to any one of claims 1 to 6, alone or in combination with one or more inert non-toxic excipients.

12. Pharmaceutical compositions containing as active ingredient 3[N-(2-chloroethyl)-N-nitrosoamido]-2-[1-(4-methylpiperazinyl)-2-oxo-2H-tetrahydro-1,3,2-oxaazaphosphorine or one of its pharmaceutically acceptable acid addition salts, alone or in combination with one or more inert non-toxic excipients.

**Claims for the Contracting State:** AT

1. Process for the preparation of novel oxaazaphosphorine derivatives corresponding to the general formula I

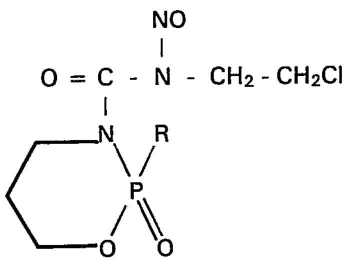

I

in which R represents:

- a straight-chain or branched-chain alkyl group containing from 1 to 6 carbon atoms,
- a cycloalkyl group containing from 3 to 7 carbon atoms,
- a phenyl group that may be substituted by an alkyl or alkoxy radical containing from 1 to 4 carbon atoms or by a halogen atom,
- a phenylalkyl group that contains from 7 to 10 carbon atoms and in which the phenyl ring may optionally be substituted by a halogen atom or an alkyl or alkoxy radical containing from 1 to 4 carbon atoms,
- a straight-chain or branched-chain alkoxy group containing from 1 to 4 carbon atoms,
- a phenoxy or thiophenoxy group,
- a mono- or di-alkylamino group that contains from 1 to 8 carbon atoms and that may optionally be substituted by one or two halogen atoms, or
- a pyrrolidin-1-yl, morpholin-4-yl or piperid-1-yl group, or a piperazin-1-yl group that is optionally substituted in the 4-position by a straight-chain or branched-chain alkyl radical containing from 1 to 4 carbon atoms, by a cycloalkyl group containing from 5 to 7 carbon atoms, or by a phenyl or phenylalkyl group that contains from 7 to 10 carbon atoms and in which the phenyl ring may be substituted by a halogen atom or a straight-chain or branched-chain alkyl or alkoxy radical containing from 1 to 4 carbon atoms,

and also, when R represents a diamino group, their pharmaceutically acceptable acid addition salts, characterised in that a phosphonic or phosphoric acid dichloride of the formula II

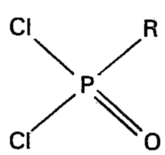

II

in which R has the meaning defined in formula I,

is condensed with3-aminopropan-1-ol in an inert solvent and in the presence of an acid acceptor at a temperature between –30 and 0°C to form a compound of the formula III

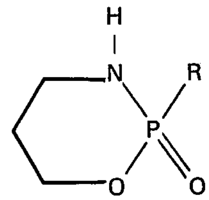

III

in which R has the meaning defined in formula I,

and which is condensed with an excess of β-chloroethyl isocyanate of the formula IV

$Cl\text{-}CH_2\text{-}CH_2\text{-}N=C=O$ IV

either directly, when R represents an amino group, or, when R is not an amino group, after previously activating the nitrogen atom of the 1,3,2-oxaazaphosphorine ring by means of butyllithium, to obtain a compound of the formula V

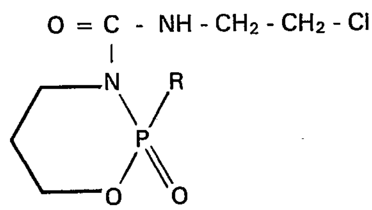

V

in which R has the meaning defined in formula I,

and which is then nitrosated in an acidic or basic medium by the action of nitrosating agents, to form a compound of the formula I,

which, when R is a diamino group, can then be converted into its pharmaceutically acceptable acid addition salt.

2. Process according to claim 2, characterised in that the nitrosation of a derivative of the formula V is carried out after previously activating the nitrogen atom of the urea.

3. Process according to claims 1 and 2, characterised in that the activation of the nitrogen atom before nitrosating a derivative of the formula V is carried out by means of butyllithium in tetrahydrofuran at a temperature of less than –60°C.

4. Process according to any one of claims 1 to 3, characterised in that the derivatives of the formula V are nitrosated by nitrosyl chloride, sodium nitrite or nitrogen tetroxide in an acidic or basic solvent.